# EUROPÄISCHE PATENTSCHRIFT

(11) **EP 3 737 223 B1**
(45) Veröffentlichungstag und Bekanntmachung des Hinweises auf die Patenterteilung: **22.12.2021**
(21) Anmeldenummer: 19701581.1
(22) Anmeldetag: 10.01.2019
(51) Int. Cl.: A01G 24/44, A01G 24/20, A01H 4/00, C12N 5/00

(54) **NÄHRMEDIUM FÜR DIE AUTOMATISIERTE PRODUKTION VON PFLANZEN**
NUTRIENT MEDIUM FOR AUTOMATED CULTIVATION OF PLANTS
MILIEU NUTRITIF POUR LA PRODUCTION AUTOMATISÉE DE PLANTES

(30) Priorität: 10.01.2018 DE 102018100485
(43) Veröffentlichungstag der Anmeldung: 18.11.2020
(73) Patentinhaber: RoBoTec PTC GmbH, 28357 Bremen (DE)
(72) Erfinder: VON RUNDSTEDT, Friederike, 28357 Bremen (DE)
(74) Vertreter: Hoener, Matthias
(86) Internationale Anmeldenummer: PCT/EP2019/050511
(87) Internationale Veröffentlichungsnummer: WO 2019/137981

(56) Entgegenhaltungen:
- US-A1- 2012 220 036

## Beschreibung

Die Erfindung betrifft ein Nährmedium für Pflanzen und daraus hergestellte Pflanzstopfen ("*in-vitro* plugs").

Im Bereich der vegetativen Pflanzenkultivierung, insbesondere der Vermehrung von fruchttragenden und ästhetischen Pflanzen, wird es immer wichtiger, eine standardisierte und keimfreie Herstellung neuer Pflanzen zu gewährleisten. Insbesondere die Verwendung von Erdsubstraten ist aufgrund einer wachsenden Internationalisierung des Handels zunehmend mit Problemen verbunden. So untersagen verschiedene Länder die Einfuhr von Pflanzen, die auf Erdsubstraten kultiviert wurden, da ihre Keimfreiheit kaum gewährleistet werden kann. Die Verwendung von Erdsubstraten ist daher häufig mit einen erhöhten Aufwand verbunden, wenn z. B. verschiedene Wachstumssubstrate für unterschiedliche Länder etabliert oder Sondergenehmigungen für ihre Einfuhr erwirkt werden müssen.

Pflanzenzüchter setzen aus diesen Gründen vermehrt auf *in-vitro* Substrate für die Pflanzenvermehrung, z. B. auf Basis von Gellan (Gellan Gum), wie z.B. in der US 2012/0220036 A1 offenbart. Die *in-vitro* Substrate enthalten in der Regel standardisierte Nährstoffmengen und können sterilisiert werden, sodass sich ihre Keimfreiheit garantieren lässt. Üblicherweise werden für die regenerative Vermehrung einzelne Sprossen von Hand auf das *in-vitro* Substrat aufgebracht. Sobald sich ein feines Wurzelwerk gebildet hat, kann dann - wiederum von Hand - die aus Spross und Substrat gebildete Pflanzeinheit weiterbehandelt werden, z. B. indem beides in ein Erdsubstrat eingebracht wird. Diese Vorgehensweise ist jedoch personal-, zeit- und kostenintensiv. Darüber hinaus ist die manuelle Bearbeitung nur in eingeschränktem Maße standardisierbar.

In der PCT/EP2017/000922 ist eine Vorrichtung beschrieben, die in der Lage ist, die oben beschriebenen Vermehrungsschritte automatisiert durchzuführen. Die Vorrichtung ergreift zunächst eine einzelne zu vermehrende Pflanze durch einen ersten Greifer. Anschließend werden die einzelnen an dem ersten Greifer hängenden Pflanzen gezielt in mehrere Klone zerschnitten und die einzelnen Klone automatisiert durch einen zweiten Greifer für die weitere Verarbeitung abtransportiert.

Um die darauf folgenden Schritte - insbesondere das Aufbringen der Klone auf einen Pflanzstopfen aus einem Nährmedium und die weitere Handhabung dieses Pflanzstopfens - ebenfalls automatisieren zu können, besteht ein Bedarf an einem Nährmedium, das einerseits fest genug ist, um die Handhabung durch Greifer einer Vorrichtung zu erlauben, und andererseits die Ausbildung eines hinreichenden Wurzelwerks nicht verhindert. Derzeit bekannte *in-vitro* Nährmedien erfüllen diese Voraussetzungen nicht, da sie keine ausreichende Stabilität aufweisen.

### Beschreibung

Gelöst wird die Aufgabe durch ein Nährmedium, das die kennzeichnenden Merkmale des Anspruchs 1 aufweist, sowie durch Pflanzstopfen, die dieses Nährmedium umfassen. Insbesondere betrifft die Erfindung in einem ersten Aspekt ein Nährmedium für die Kultivierung von Pflanzen, wobei es (a) 3 g/l bis 18 g/l Agar; und (b) 0,5 g/l bis 3 g/l Carrageen und (c) eine zusätzliche formstabilisierende Komponente umfasst. Überraschenderweise wurde im Rahmen der Erfindung festgestellt, dass diese Mischung aus den Geliermitteln Agar und Carrageen eine ausreichende Festigkeit für die Handhabung durch maschinelle Greifer aufweist und gleichzeitig die Ausbildung des notwendigen Wurzelwerks zulässt, obwohl die einzelnen Geliermittel für sich in vergleichbaren Konzentrationen keinen ausreichenden Geliereffekt erzeugen würden. Das erfindungsgemäße Nährmedium eignet sich damit sowohl für die manuelle als auch für die automatische Kultivierung und Vermehrung von Pflanzen. In dem erfindungsgemäßen Nährmedium können alle wichtigen Schritte einer Kultivierung durchgeführt werden, inklusive der Entwicklung, der Vermehrung, dem Schutz und der Optimierung der Pflanzen.

Gegenüber den derzeit verwendeten Nährmedien und Verfahren zur Pflanzenkultivierung zeichnet sich die Erfindung insbesondere dadurch aus, dass die Pflanzen schneller wachsen, da sie in einem mit dem erfindungsgemäßen Nährmedium hergestellten Pflanzstopfen nicht in ihrem Wachstum gehemmt werden. Die derart kultivierten Pflanzen entwickeln sich uniform und zu robusten Pflanzen. Darüber hinaus gehen die erfindungsgemäß automatisierbaren Kultivierungsverfahren mit einer guten Planbarkeit von Kultur- und Verkaufsparametern, Kosten- und Zeiteinsparungen durch Automatisierung, reduzierte Handarbeit, gesicherte Pflanzengesundheit und höheren Anwachsraten einher. Die erfindungsgemäßen Nährmedien und Pflanzstopfen sind weltweit exportierbar, da sie steril und krankheitsfrei sind und Pflanzengesundheit damit zertifizierbar ist. Die jungen Pflanzen können mittels der Pflanzstopfen und einem korrespondierenden Pflanztablett auch vor Austrocknung und Beschädigung geschützt werden. Schließlich können die Pflanzen in den Pflanzstopfen auch leichter an unsterile Gewächshausbedingungen nach dem Pikieren angepasst werden, sodass Ausfälle reduziert werden.

Das erfindungsgemäße Nährmedium ist insbesondere ein für die vegetative Vermehrung von Pflanzen geeignetes Medium. Gleichzeitig kann das Medium jedoch auch für die generative Vermehrung, insbesondere die Aussaat, von Pflanzen verwendet werden. Im Gegensatz zu einer generativen Vermehrung ist die vegetative Vermehrung ungeschlechtlich und auf die Pflanzenanzucht aus Pflanzenteilen, z. B. Stecklingen, angewiesen. Fachleuten ist bekannt, dass Nährmedien - neben den hier als wesentlich beschriebenen Bestandteilen - weitere Zusatzstoffe, wie Nährstoffe und dergleichen, enthalten. Diese sind auf die jeweilige Pflanzenart, den Kultivierungszweck und die Kultivierungsart (vegetativ / generativ) abzustimmen und daher variabel. Geeignete Zusatzstoffe und Zusatzstoffkombinationen sind an anderer Stelle hierin beschrieben.

Die erfindungsgemäß kultivierbaren und vermehrbaren Pflanzen können jegliche Pflanzen sein, die auf einem Substrat wachsen können, das die Geliermittel Agar und Carrageen umfasst. In besonders bevorzugten Ausführungsformen sind die erfindungsgemäßen Produkte und Verfahren für die Kultivierung von Zierpflanzen, Stauden und/oder Gehölzen geeignet. Bevorzugte Zierpflanzen umfassen z. B. *Phalaenopsis* (Orchideen), *Anthurium* und *Spathiphyllum.* Bevorzugte Stauden umfassen z. B. *Echinacea, Helleborus* und *Heuchera.* Bevorzugte Gehölze umfassen z. B. *Lycium, Paulownia* und *Vaccinium.* Es versteht sich, dass die Verwendung der erfindungsgemäßen Produkte und Verfahren nicht auf diese Pflanzen beschränkt ist.

Der Begriff "Kultivierung" bezeichnet die Schaffung und Aufrechterhaltung von Bedingungen, die ein Wachstum von Pflanzen gewährleisten, d.h. fördern und/oder erlauben. Dies umfasst Bedingungen, welche die Vermehrung gewährleisten. Bevorzugt werden die erfindungsgemäßen Verfahren und Produkte für die Vermehrung von Pflanzen eingesetzt, d. h. die Kultivierung eines Pflanzensprosses aus einem Samen oder die Kultivierung eines Pflanzensprosses aus einem Klon.

Wie erwähnt, umfasst das Nährmedium 3 g/l bis 18 g/l Agar. Bevorzugt umfasst das Nährmedium von 5 g/l bis 12 g/l Agar, stärker bevorzugt von 5,8 g/l bis 9,5 g/l. In einer Ausführungsform umfasst das Nährmedium Agar in einer Konzentration von 5,8 g/l bis 7 g/l. So kann Agar z. B. in einer Konzentration von etwa 6,4 g/l in dem Nährmedium enthalten sein.

Agar (auch: Agar-Agar) ist ein strukturelles Kohlenhydrat in den Zellwänden bestimmter Algen, das Galactose-Polymere umfasst und Gallerte bilden kann. Quellen zur Gewinnung von Agar und Verfahren zur Herstellung von Agar sind Fachleuten bekannt. So kann Agar z. B. aus den Zellwänden einiger Algenarten gewonnen werden, z. B. aus Rotalgen. In Agar ist der starke Gelbildner Agarose für die Gelierfähigkeit verantwortlich. In der EU ist Agar als Lebensmittelzusatzstoff unter der Nummer E 406 zugelassen. Während Agar bereits bei 45°C geliert, ist es sehr temperaturbeständig, sodass es auch bei hohen Temperaturen sterilisiert werden kann. Darüber hinaus ist es auch mehrfach nacheinander sterilisierbar ohne wesentlich an Festigkeit zu verlieren. Agar ist in ausreichenden Mengen im Handel erhältlich (z. B. als CERO Agar Agar powder, Type 8925 X; CERO Agar Agar Gracellaria Powder, Type 8925 Q; 1-Plant Agar 1200 g/cm²; I.A.-Mikro Agar 880 g/cm²; Vitro Agar 1200-900 g/cm²; Vitro A1 Agar 1200 g/cm²; Gelrite; Gellan Gum Typ 2). Vorzugsweise weist der Agar eine Gelstärke (bestimmt nach Nikkan-Kobe Test; 1,5%, 15h) von 800 bis 1300 g/cm² auf, bevorzugt von 800 bis 1000 g/cm², stärker bevorzugt 850 bis 960 g/cm², noch stärker bevorzugt 890 bis 930 g/cm², z. B. 910 g/cm². Verfahren zur Bestimmung der Gelstärke sind Fachleuten bekannt und umfassen den Nikkan-Kobe Test.

Ferner umfasst das Nährmedium 0,5 g/l bis 3 g/l Carrageen. Bevorzugt umfasst das Nährmedium 1 g/l bis 2,2 g/l Carrageen, stärker bevorzugt 1,3 g/l bis 1,9 g/l Carrageen. So kann Carrageen z. B. in einer Konzentration von etwa 1,6 g/l in dem Nährmedium enthalten sein.

Carrageen ist ebenfalls ein Geliermittel aus langkettigen Kohlenhydraten die in Rotalgenzellen (Irish Moos Chondrus crispus) vorkommen (Synonym: Dänisch Agar). In der EU ist Carrageen als Lebensmittelzusatzstoff unter der Nummer E 407 zugelassen. Carrageen ist in ausreichenden Mengen im Handel erhältlich (z. B. als CEROGEL Carrageenan, Type 8886). Vorzugsweise weist das Carrageen eine Gelstärke (bestimmt nach Nikkan-Kobe Test; 1,5%, 20°C) von 400 bis 1000 g/cm² auf, stärker bevorzugt 500 bis 900 g/cm², noch stärker bevorzugt 600 bis 800 g/cm². Das Carrageen kann unmittelbar nach seiner Isolierung aus Rotalgenextrakten in dem erfindungsgemäßen Nährmedium verwendet werden. Da sich innerhalb des Carrageen-Extrakts insbesondere die Kappa-Fraktion durch eine gute Gelierfähigkeit auszeichnet, umfasst das Carrageen bevorzugt einen hohen Anteil an Kappa-Carrageen (κ-Carrageen). So kann das Carrageen z. B. 80% oder mehr, bevorzugt 90% oder mehr Kappa-Carrageen umfassen. Bevorzugt ist das in dem erfindungsgemäßen Nährmedium verwendete Carrageen Kappa-Carrageen.

In einer bevorzugten Ausführungsform umfasst das Medium von 3 bis 10 g/l Agar und 0,5 bis 3 g/l Carrageen, stärker bevorzugt von 5 g/l bis 8 g/l Agar und 1 g/l bis 2,2 g/l Carrageen. So kann das Medium z. B. etwa 6,4 g/l Agar und 1,6 g/l Carrageen umfassen. Überraschenderweise erzeugen die beiden Komponenten Agar und Carrageen in diesen Konzentrationen in synergistischer Weise bereits eine für die automatische Handhabung geeignete Gelstärke.

Das Medium kann Agar und Carrageen beispielsweise in einem Verhältnis im Bereich von 6:1 bis 2:1 umfassen, vorzugsweise im Bereich von 5:1 bis 3:1, stärker bevorzugt im Bereich von 4:1 bis 3,5:1.

In einer bevorzugten Ausführungsform ist das Medium ein steriles Medium. Verfahren zur Sterilisierung sind Fachleuten bekannt. Das Nährmedium kann z. B. durch Dampfdrucksterilisation (Autoklavieren), Mikrowellensterilisation, Bestrahlung oder Sterilfiltration sterilisiert werden. Es versteht sich, dass das letztgenannte Verfahren nur zur Sterilisierung des Mediums in noch flüssiger Form geeignet ist. Bevorzugt werden Sterilisation und Gelierung/Verfestigung des Nährmediums miteinander verbunden. Daher sind die Dampfdrucksterilisation und Mikrowellensterilisation besonders bevorzugt.

Das Nährmedium ist bevorzugt frei von Bestandteilen, die zur mikrobiellen Kontaminierung der Pflanzen führen könnten. Das Nährmedium ist daher vorzugsweise frei von Erden (erdfrei). Der Begriff "Erden" umfasst dabei z. B. Humus, Blumenerde, Torf und dergleichen.

Fachleuten ist bekannt, dass die Geliermittel Agar und Carrageen erst ab bestimmten Temperaturen gelieren und dem Nährmedium damit seine für den (automatisierten) Einsatz als Pflanzstopfen benötigte festere Form geben. Das erfindungsgemäße Nährmedium kann daher eine flüssige oder eine festere bzw. feste Form aufweisen. Geeignete Gelstärken, die hierin als "fest" gelten sollen, sind an anderer Stelle hierin definiert. Während das Nährmedium in fester Form erfindungsgemäß bevorzugt ist, sind auch flüssige Nährmedien von der Erfindung umfasst. So ist es z. B. denkbar, dass das Nährmedium in flüssiger Form transportiert wird. Flüssiges Nährmedium kann auch zur Herstellung der Pflanzstopfen verwendet werden, z. B. durch Gießen in entsprechende Formen.

Agar und Carrageen gelieren bei Temperaturen ab 35-41 °C (Agar). Dies schließt allerdings nicht aus, dass höhere Temperaturen für die Gelierung des Nährmediums verwendet werden können, z. B. zur gleichzeitigen Sterilisierung. Fachleute sind in der Lage, geeignete Geliertemperaturen zu ermitteln. Im Gegensatz zu anderen Geliermitteln ist die erfindungsgemäße Mischung aus Agar und Carrageen ohne wesentlichen Qualitätsverlust mehrfach erhitzbar und damit bei Bedarf z. B. auch mehrfach sterilisierbar.

Das erfindungsgemäße Nährmedium zeichnet sich dadurch aus, dass es eine Gelstärke aufweist, die es erlaubt, mit dem Nährmedium erzeugte Pflanzstopfen maschinell zu greifen und zu bewegen und gleichzeitig die Pflanzen nicht daran hindert, ein hinreichend dichtes Wurzelsystem auszubilden. Das Nährmedium weist insbesondere eine Gelstärke von 400 bis 1200g/cm² auf, vorzugsweise 600 bis 1000g/cm².

Das erfindungsgemäße Nährmedium umfasst eine zusätzliche formstabilisierende Komponente. Diese formstabilisierende Komponente trägt neben dem Agar und dem Carrageen dazu bei, die Form des Pflanzstopfens auch bei mechanischer Beanspruchung, wie dem automatisierten Versetzen des Stopfens, und über einen längeren Kultivierungszeitraum zu wahren. Die formstabilisierende Komponente können/kann eine wasserlösliche und/oder eine wasserunlösliche Komponente sein.

In bevorzugten Ausführungsformen ist die formstabilisierende Komponente eine wasserunlösliche Komponente. Diese Komponente stabilisiert einen Pflanzstopfen, der aus dem erfindungsgemäßen Nährmedium hergestellt ist und stellt sicher, dass das innerhalb eines Pflanzstopfens gebildete Wurzelwerk nicht in sich zusammenfällt, wenn der Stopfen mit Wasser gegossen und die Geliermittel dadurch mit der Zeit aufgelöst werden. Dies passiert regelmäßig im Laufe der Kultivierungszeit, wenn ein Pflanzstopfen unter Gewächshausbedingungen in eine Vertiefung in dem nächst größeren Erdsubstrat eingesetzt und dort begossen wird. Die wasserunlösliche Komponente ist daher eine durch Wasser nicht auflösbare Komponente, insbesondere ein von Wasser nicht auflösbares Netzwerk.

Die wasserunlösliche Komponente liegt in dem Pflanzstopfen (und optional auch bereits in dem flüssigen Nährmedium) in Form eines Netzwerks vor. Dies gilt gleichermaßen für die unten näher beschriebene wasserlösliche Komponente. Das Netzwerk bildet ein Gerüst zur Stützung eines gebildeten oder zu bildenden Wurzelwerks. Das Netzwerk kann innerhalb des Nährmediums bzw. Pflanzstopfens aus der wasserunlöslichen Komponente entstehen. Alternativ kann das Netzwerk dem Nährmedium bzw. Pflanzstopfen bereits in Form eines Netzwerks zugegeben werden. Das Netzwerk wird in der Regel ein Außenvolumen einnehmen, das dem eines Pflanzstopfens im Wesentlichen entspricht, z. B. höchstens 40%, vorzugsweise höchstens 30%, stärker bevorzugt höchstens 20%, kleiner ist. Dabei weist das Netzwerk Poren und vernetzte Aushöhlungen auf. Innerhalb der Poren und vernetzten Aushöhlungen kann sich ein Wurzelwerk bilden.

Die wasserunlösliche Komponente ist vorzugsweise ausgewählt aus der Gruppe bestehend aus Pflanzenteilen, Kunststoffen, Mineralstoffen, Mischungen derselben und daraus hergestellten Materialien.

Geeignete Pflanzenteile sind z. B. Teile von Jute, Luffa, Flachs, Banane, Kokos, Spaghnummoos, Arganfruchtschalen, Rinde, Kork, Hanf, und weitere, insbesondere faserige oder faserhaltige, Pflanzenteile bzw. Pflanzen; wobei faserige oder faserhaltige Teile von Jute, Hanf, Flachs und Kokos besonders bevorzugt sind, d.h. Jute-, Hanf-, Flachs- und Kokosfasern.

Geeignete Kunststoffe umfassen z. B. Polyurethan (z. B. in Form von Polyurethanschaum) und Biokunststoffe, wie Polylactide (PLA; Polymilchsäuren).

Polyurethan, vorzugsweise Polyurethanschaum, wird in der Regel aus einer Polyol- und einer Isocyanatkomponente hergestellt, die gemischt und innerhalb einer Form kalt aufgeschäumt werden. Anschließend kann der verfestigte Polyurethan(schaum) in kleinere Teile in Stopfenform und -größe geschnitten werden. Diese Teile können in einer in etwa form- und größenkomplementären Form mit dem flüssigen, erfindungsgemäßen Nährmedium übergossen werden. Geeignete Polyurethanschäume sind Fachleuten bekannt und kommerziell erhältlich (z.B. Oasis, Smithers-Oasis Germany GmbH, D-67269 Grünstadt, und BVB Sublime, BVB Substrates NL-2678 PS De Lier). Es hat sich herausgestellt, dass Polyurethanschäume mit einer Porengröße von 2,00 bis 0,05 cm eine besonders gute Verwurzelung und gleichzeitige Stabilisierung ermöglichen, weshalb solche Polyurethanschäume erfindungsgemäß besonders bevorzugt sind. Dies gilt gleichermaßen für andere im Rahmen der Erfindung verwendete formstabilisierende Komponenten. Der Polyurethanschaum ist vorzugsweise ein Weichschaum. Weichschäume haben den Vorteil, dass sie nach dem Trocknen flexibel (weich) bleiben und nicht starr werden. Auch dies gilt gleichermaßen für andere formstabilisierende Komponenten, d.h. die formstabilisierende Komponente ist vorzugsweise ein Schaum, der Poren aufweist, vorzugsweise mit einer Porengröße von 2,00 bis 0,05 cm.

Für die Erfindung geeignete Polylactide (PLAs) weisen vergleichbare Eigenschaften zu den oben geschilderten Polyurethanen auf. Solche PLAs sind Fachleuten bekannt. Die PLAs weisen darüber hinaus eine hohe Biokompatibilität auf und können biologisch abbaubar ausgebildet werden. Das Polylactid ist somit vorzugsweise ein biologisch abbaubares PLA. Das Polylactid kann ferner ein Polylactid sein, dass mittels 3D-Drucks druckbar ist. Auf diese Weise kann beispielsweise mittels 3D-Drucks ein formstabilisierendes Polylactidgerüst gedruckt und anschließend mit dem erfindungsgemäßen Nährmedium übergossen werden.

Geeignete Mineralstoffe umfassen unter anderem Vermiculit, andere Siliciumverbindungen, Tonmineralien und Perlite. Aus den vorgenannten Stoffgruppen hergestellte Materialien, die für das erfindungsgemäße Nährmedium eingesetzt werden können, sind z. B. Steinwolle, Zellstoff und dergleichen.

Von Pflanzen werden bevorzugt getrocknete Pflanzenteile verwendet, z. B. getrocknete Teile der Frucht (Luffa) oder dergleichen. Bevorzugt ist die wasserunlösliche Komponente Luffa. Diese Bestandteile sind reich an Zellulose. Es versteht sich, dass getrocknete Pflanzenteile vor ihrer Verwendung auf geeignete Weise zerkleinert werden können, z. B. durch mörsern. Wie oben beschrieben kann die wasserunlösliche Komponente aber auch in einer unzerkleinerten Form, in Form eines Netzwerks direkt verwendet werden. So kann z. B. ein Teil aus einer getrockneten Luffa-Frucht herausgetrennt werden, welches in etwa das Außenvolumen eines Pflanzstopfens aufweist. Das Teil kann anschließend in einer in etwa form- und größenkomplementären Form mit dem flüssigen Nährmedium übergossen werden. Dabei läuft das flüssige Nährmedium in die Poren und vernetzte Aushöhlungen in dem Netzwerk und kann anschließend durch Erhitzung dort verfestigt werden. Alternativ können Pflanzenfasern, wie Jute-, Hanf-, Flachs- oder Kokosfasern, beispielsweise durch Verknäulen der Fasern in eine Stopfenform gebracht werden und anschließend, wie oben beschrieben, in einer Form mit dem flüssigen Nährmedium übergossen werden.

Allen vorgenannten und erfindungsgemäß geeigneten wasserunlöslichen Komponenten ist gemein, dass sie in der Lage sind, die Struktur eines Pflanzstopfens auch nach dem Auflösen der gelierenden Mittel Agar und Carrageen aufrecht zu erhalten. Die wasserunlöslichen Komponenten dienen also dazu, das gebildete Wurzelwerk zu stabilisieren. Besonders geeignet für diesen Zweck sind Komponenten, z. B. Verbindungen oder Mischungen von Verbindungen, die faserhaltig sind oder Fasern bilden. Die Komponenten, insbesondere faserhaltige Komponenten, werden in der Regel innerhalb eines Pflanzstopfens ein Netzwerk bilden oder bereits in Form eines (endogenen) Netzwerks vorliegen. Mit anderen Worten ist die wasserunlösliche Komponente ein Netzwerk oder ein Netzwerkbilder. Durch das stabilisierende Netzwerk ist die wasserunlösliche Komponente in der Lage, nach dem Auflösen und Entfernen (z. B. Abfließen) der wasserlöslichen Geliermittel Agar und Carrageen, ein vollständiges Zusammenfallen des Pflanzstopfens und des Wurzelwerks zu verhindern.

Bevorzugt wird das äußere Volumen des Pflanzstopfens bei einem Auflösen und Entfernen der Geliermittel Agar und Carrageen aufgrund einer stabilisierenden Wirkung der wasserunlöslichen Komponente um weniger als 60%, vorzugsweise weniger als 40%, stärker bevorzugt um weniger als 20% reduziert. Dies bedeutet, dass ein Pflanzstopfen-Volumen von z. B. 10 cm³ (100%) durch die wasserunlösliche Komponente so stabilisiert wird, dass es sich durch die Zugabe von Wasser und/oder das Auflösen und Entfernen der Geliermittel Agar und Carrageen höchstens auf ein Volumen von 40 cm³ (40% des Ursprungsvolumens) reduziert, vorzugsweise 60 cm³ (60% des Ursprungsvolumens), stärker bevorzugt 80 cm³ (80% des Ursprungsvolumens).

"Wasserunlöslich" bedeutet im Zusammenhang mit der Erfindung, dass Wasser keine oder nur einen geringen Einfluss auf die Integrität der Komponente und daraus gebildeter Strukturen in dem Pflanzstopfen haben. Dies schließt selbstverständlich nicht aus, dass die Komponente und daraus gebildete Strukturen ihre Form durch den Verlust von anderen wasserlöslichen Komponenten (wie Agar und Carrageen) in dem Pflanzstopfen ändern können. So kann z. B. durch den Wegfall von Agar und Carrageen ein gebildetes Netzwerk durch den Einfluss der Schwerkraft leicht (d.h. in den zuvor genannten volumetrischen Grenzen) zusammensacken. Ferner schließt der Begriff auch nicht aus, dass die Komponente durch Wasserkontakt quellen kann, solange die Netzwerkstruktur im Wesentlichen (stabilisierend und in den angegebenen volumetrischen Grenzen) erhalten bleibt.

Es versteht sich, dass das Netzwerk durch andere mechanische oder biologische Einflüsse stärker komprimiert werden kann. Da - gerade in unsterilen Gewächshausbedingungen - davon auszugehen ist, dass das Netzwerk durch solche Einflüsse im Laufe der Zeit stärker betroffen wird und auch nicht mehr zur Stabilisierung erforderlich ist, sobald sich das Wurzelwerk in ausreichendem Maße mit dem unsterilen Erdsubstrat verbunden hat, das den Pflanzstopfen umgibt, betreffen die obigen Angaben zur Stabilisierung durch das Netzwerk, lediglich die ersten sechs Wochen nach dem Transfer eines Pflanzstopfens in ein Erdsubstrat.

Alternativ oder zusätzlich zu der oben beschriebenen wasserunlöslichen, formstabilisierenden Komponente, kann das Nährmedium auch eine formstabilisierende wasserlösliche Komponente umfassen. Geeignete wasserlösliche Komponenten umfassen z. B. Hydroxyalkylcellulose und Gelatine, insbesondere Gelatine mit kolloidalem Silber. Die Hydroxyalkylcellulose ist vorzugsweise ausgewählt aus der Gruppe bestehend aus Hydroxymethylcellulose und Hydroxyethylcellulose, wobei ersteres besonders bevorzugt ist.

Geeignete Herstellungsverfahren für Hydroxyalkylcellulosen sind Fachleuten bekannt. So ist beispielsweise Hydroxyethylcellulose (HEC) ein Reaktionsprodukt alkalisierter und durch kontrollierten oxidativen Kettenabbau gereifter Cellulose mit Ethylenoxid. Hierbei können nicht nur die drei freien OH-Gruppen der Glucose-Einheit, bevorzugt die primäre Hydroxylgruppe am C-6-Atom, reagieren, sondern auch die endständige OH-Gruppe am glykolischen Substituenten. Deshalb ist der so genannte molare Substitutionsgrad (MS), die Anzahl der pro Glucose-Einheit umgesetzten Ethylenoxid-Moleküle, das Zwei- bis Fünffache des sogenannten durchschnittlichen Substitutionsgrades (DS). Unter dem DS versteht man die Anzahl der derivatisierten Hydroxylgruppen pro Glucose-Einheit der Cellulose. Höhe und Gleichmäßigkeit der Substituentenverteilung auf der Polymerkette bestimmen die Solvatation und damit die Möglichkeit zur Herstellung nahezu schwebeteilchenfreier Lösungen und Hydrogele. Die unterschiedlich stark verdickend wirkenden HEC-Typen werden bei der Reifung der Cellulose im Produktionsprozess erzeugt. Geeignete HEC sind Fachleuten bekannt und kommerziell erhältlich und umfassen beispielsweise PHRIKOLAT HEC 100, eine hochviskose Hydroxyethylcellulose mit 4.500 - 6.500 mPas in 1 %iger Lösung (Brookfield). Weitere geeignete Hydroxyalkylcellulosen umfassen z. B. Methyl Cellulose, Sigma, Prod. Nos. M0262, M0387, M0512, M6385 and M7140, CAS NUMBER: 9004-67-5; Synonym: Methylcellulose A, Methylcellulose ether. Die Hydroxyalkylcellulosen weisen vorzugsweise eine Viskosität von 400 bis 10.000 mPa·s in 2 %iger wässriger Lösung auf. Erfindungsgemäß weist die Cellulose Alkoxy-Substitutionen, vorzugsweise Methoxy-Substitutionen, von 27,5 bis 31,5 Gew.-% auf.

Die Hydroxyalkylcellulose kann dem erfindungsgemäßen flüssigen Nährmedium hinzugefügt werden und gemeinsam mit dem Nährmedium in der gewünschten Form und Größe verfestigt werden. Bevorzugt umfasst das Nährmedium Hydroxyalkylcellulose in einer Konzentration von 5 bis 15%.

Ebenfalls zur Stabilisierung eingesetzt werden kann Gelatine, vorzugsweise ein Gelatineschaum. Hierbei ist es insbesondere bevorzugt Gelatineteile zu verwenden, die eine geringere Größe aufweisen, als ein hierin definierter Pflanzstopfen, und diese mit dem erfindungsgemäßen Nährmedium zu übergießen. Verfahren zur Herstellung geeigneter Gelatineteile, z.B. Gelatinewürfel, sind Fachleuten bekannt. Darüber hinaus sind Gelatineteile auch kommerziell erhältlich. Es ist angedacht, vorzugsweise Gelatineteile zu verwenden, die kolloidales Silber enthalten und dadurch eine zusätzliche sterilisierende Wirkung entfalten. Entsprechende Gelatinewürfel sind beispielsweise als "Gelatamp" (Roeko; Coltene, Gelatineschwamm mit 5 % kolloidalem Silber, y-steril) und "Gelita-Spon^{®}" (Gelita medical; z. B. Cube - Würfel, 10 x 10 x 10, 50, GS-310, Art. 00715118, ohne Silberzusatz, geschäumtes Gel) erhältlich.

Wie oben erwähnt, umfasst das Nährmedium weitere Zusatzstoffe, wie z. B. Nährstoffe. Das Nährmedium kann z. B. Nährstoffe, wie z. B. Makro- und Mikronährstoffe; Vitamine, Phytohormone, weitere Geliermittel, Zucker und/oder weitere enthalten. Alle Zusatzstoffe fördern bzw. unterstützen das Wachstum der verschiedenen Pflanzenarten. Dies kann auf unterschiedliche Weise geschehen. So können die Zusatzstoffe das Pflanzenwachstum unmittelbar unterstützen, z. B. indem sie Bausteine für die Ausbildung von Zellen und dergleichen bereitstellen, oder sie können das Pflanzenwachstum nur indirekt unterstützen, z. B. indem sie das Wachstum von konkurrierenden Organismen, wie Bakterien, verhindern oder eindämmen. Die Zusatzstoffe werden jeweils in Abhängigkeit von der zu kultivierenden Pflanzenart ausgewählt und kombiniert.

Während es wie oben beschrieben möglich ist, Zusatzstoffe zu dem Nährmedium hinzuzugeben, die das Wachstum von konkurrierenden Organismen, wie Bakterien, Pilzen und dergleichen, verhindern oder eindämmen, wurde überraschenderweise festgestellt, dass die hierin definierte Mischung aus den Geliermitteln Agar und Carrageen das Bakterienwachstum ebenfalls hemmt. Der Zusatz entsprechender Zusatzstoffe ist somit erfindungsgemäß nicht zwingend erforderlich.

Geeignete Nährstoffe umfassen Makronährstoffe, wie Stickstoff (N), Phosphor (P), Kalium (K), Calcium (Ca), Magnesium (Mg) und Schwefel (S), wobei die Makronährstoffe vorzugsweise in Form von chemischen Verbindungen in dem Nährmedium enthalten sind, die den jeweiligen Makronährstoff enthalten und für die Pflanzen verfügbar machen. Das Nährmedium umfasst z. B. Makronährstoffe in Form von KNO₃, NH₄NO₃, MgSO₄ x 7 H₂O, KH₂PO₄ und/oder CaCl₂ x 2 H₂O. Die Konzentration beträgt von 100 bis 2000 ppm.

Geeignete Nährstoffe umfassen ferner Mikronährstoffe, wie Bor (B), Eisen (Fe), Iod (I), Kobalt (Co), Kupfer (Cu), Mangan (Mn), Molybdän (Mo), Natrium (Na) und Zink (Zn), wobei die Mikronährstoffe vorzugsweise in Form von chemischen Verbindungen in dem Nährmedium enthalten sind, die den jeweiligen Makronährstoff enthalten und für die Pflanzen verfügbar machen. Das Nährmedium umfasst z. B. Makronährstoffe in Form von MnSO₄ x H₂O, ZnSO₄ x 7 H₂O, H₃BO₃, Na₂MoO₄ x 2 H₂O, CuSO₄ x 5 H₂O, KJ und CoCl₂ x 6 H₂O und NaFe EDTA. Die Konzentration im Nährmedium beträgt von 0,01 bis 50 ppm.

Geeignete Vitamine und Vitamin-ähnliche Substanzen, die das Nährmedium umfassen, sind z. B. Thiamin, Nicotinsäure, Pyridoxin, Glycin und Myo-Inositol. Die Konzentration im Nährmedium beträgt von 0,01 bis 200 ppm.

Phytohormone steuern bestimmte Wachstumsprozesse der Pflanzen, sodass ihre Beimischung und Konzentration abhängig von der Pflanzenart und dem Wachstumszweck (Wurzelbildung, Sprossbildung, Verzweigung, Streckung etc.) ausgewählt wird. Fachleute sind in der Lage, eine angemessene Auswahl an Phytohormonen, wie auch von den anderen hierin definierten Zusatzstoffen, zu treffen und jeweils geeignete Konzentrationen zu wählen. Das Nährmedium kann z. B. Phytohormone aus folgende Wirkstoffgruppen umfassen: Abcisinsäure, Auxine, Cytokinine, Gibberelline. Das oder die Phytohormone sind in dem Nährmedium z. B. enthalten als Indol-3-essigsäure (IAA), 4-(indo-3-yl)buttersäure (IBA), 1-Naphthylacetic-Acid (NAA), 6-Benzylaminopurin (BAP), Kinetin (KIN), Zeatin (ZEA) oder 2-lsopentenyl-Adenin (2iP). Die Konzentrationen im Nährmedium betragen von 0,001 bis 50 ppm.

Das Nährmedium kann ferner Zucker umfassen. Der Zucker wird vorzugsweise in Form von Saccharose, Glukose oder/und Fructose zugefügt. Die Konzentrationen im Nährmedium liegen bei 1 bis 50 g/L Nährmedium.

Selbstverständlich kann vorgesehen sein, dass das Nährmedium einen bestimmten pH Wert aufweist, z. B. einen pH-Wert bei dem die jeweilige Pflanze optimal gedeiht.

Fachleute können je nach Pflanzenart und Einsatzzweck weitere Zusatzstoffe auswählen und dem Nährmedium beifügen.

In einem verwandten Aspekt betrifft die Erfindung Pflanzstopfen für die Kultivierung von Pflanzen, dadurch gekennzeichnet, dass der Pflanzstopfen ein erfindungsgemäßes Nährmedium umfasst.

Pflanzstopfen (häufig bezeichnet als *in-vitro*-plugs) sind in der Regel relativ kleine Formkörper aus einem Nährmedium, die der Kultivierung und Vermehrung von Pflanzen in einem sehr frühen Entwicklungsstadium dienen. Pflanzstopfen weisen in der Regel eine Konsistenz auf, die einen manuellen oder maschinellen Transfer der Stopfen in andere Kultivierungsgefäße oder Transport- oder Bearbeitungseinheiten ermöglicht. Durch ihre geringe Größe und Transferierbarkeit können sich Pflanzstopfen für Medium- und Hochdurchsatzverfahren in der Pflanzenkultivierung eignen und können auch für den platzsparenden Transport von Pflanzen in einem frühen Entwicklungsstadium verwendet werden.

Die erfindungsgemäßen Pflanzstopfen eignen sich sowohl für die manuelle als auch für automatisierte oder teilweise automatisierte und maschinelle Kultivierung von Pflanzen. Bevorzugt sind die Pflanzstopfen für die automatisierte oder teilweise automatisierte Kultivierung von Pflanzen. "Automatisiert" bedeutet in diesem Zusammenhang und sinngemäß nach DIN V 19233, dass die Kultivierung durch eine Einrichtung ausgeführt wird, die derart ausgerüstet ist, dass die Einrichtung ganz oder teilweise ohne Mitwirkung eines Menschen bestimmungsgemäß arbeitet (d.h. einen Fortschritt in der Kultivierung von Pflanzen erzielt). Mit anderen Worten arbeitet die Einrichtung autonom. Bei der automatisierten oder teilweise automatisierten Kultivierung von Pflanzen wird insbesondere ein Pflanzenspross bzw. Pflanzenklon automatisiert auf einen Pflanzstopfen aufgebracht; die aus Pflanzenteil und Pflanzstopfen gebildete Pflanzeinheit wird in eine andere Vorrichtung, einen anderen Vorrichtungsteil und/oder ein Behältnis überführt; und/oder die Pflanzeinheit wird in einen größeren Pflanzstopfen oder ein Erdsubstrat überführt, wobei der größere Pflanzstopfen ein erfindungsgemäßer Pflanzstopfen oder eine andere Art von Pflanzstopfen sein kann.

Das automatisierte "Aufbringen" eines Pflanzensprosses oder -klons auf einen Pflanzstopfen umfasst verschiedene Aufbringtechniken, z. B. das Aufbringen durch Auflegen oder das Aufbringen durch Andrücken. Es ist ebenfalls denkbar, einen Pflanzenspross oder -klon durch Einschieben in eine vorher in den Pflanzstopfen eingebrachte Vertiefung (z. B. einen Schlitz) auf einen Pflanzstopfen aufzubringen. Dabei ist der Pflanzenspross bzw. Pflanzenklon in der Regel so ausgerichtet, dass seine Blätter von dem Pflanzstopfen wegweisen und sein Stängel mit dem Pflanzstopfen in direktem Kontakt ist.

Der Pflanzstopfen ist vorzugsweise herstellbar nach dem hierin an anderer Stelle beschriebenen Verfahren zur Herstellung eines Pflanzstopfens.

Wie beschrieben, umfasst der Pflanzstopfen ein erfindungsgemäßes Nährmedium. Da der Pflanzstopfen für seine Handhabung und Verwendung eine feste Form aufweisen muss, ist das in dem Pflanzstopfen enthaltene Nährmedium fest, ebenso wie der Pflanzstopfen selbst. Wie an anderer Stelle bereits erläutert, bedeutet "fest" im Sinne der Erfindung, dass der Pflanzstopfen eine ausreichende Festigkeit für eine maschinelle Handhabung aufweist. Gleichzeitig ist der Pflanzstopfen auch nicht so fest, dass die pflanzliche Wurzelbildung verhindert wird. Entsprechende Parameter für eine geeignete Festigkeit sind bereits an anderer Stelle für das Nährmedium definiert und können in gleicher Weise für den Pflanzstopfen gelten.

Der Pflanzstopfen besteht vorzugsweise aus dem Nährmedium. Es ist aber auch denkbar, dass der Pflanzstopfen nur zu 70% oder mehr, vorzugsweise 80% oder mehr, stärker bevorzugt zu 90% oder mehr aus dem Nährmedium besteht.

Die Größe des Pflanzstopfens wird an die Größe der Wurzeln und des Wurzelgeflechts einer sich entwickelnden Pflanze und den Verwendungszweck angepasst. Dabei ist es denkbar, sehr kleine Pflanzstopfen auszubilden, die der Kultivierung z. B. bis zu einer Bewurzelungsphase/Gewebekulturphase in *Stage III* dienen. Daneben können auch größere Pflanzstopfen hergestellt werden, z. B. solche, die Aussparungen für kleinere Pflanzstopfen enthalten und in einem "Plug-In-Plug"-System verwendet werden können. Der Pflanzstopfen kann somit z. B. eine Größe von 0,125 cm³ oder mehr, vorzugweise 1 cm³ oder mehr aufweisen. Mit anderen Worten weist der Pflanzstopfen vorzugsweise ein Volumen von 0,125 cm³ bis 27 cm³ auf, stärker bevorzugt von 1 cm³ bis 10 cm³. Da die erfindungsgemäßen Pflanzstopfen besonders für die automatisierte Hochdurchsatz-Kultivierung geeignet sind, können die Pflanzstopfen in einigen Ausführungsformen besonders klein sein. Daher weist der Pflanzstopfen in einigen Ausführungsformen ein Volumen von 27 cm³ oder weniger, 16 cm³ oder weniger, bevorzugt 10 cm³ oder weniger, stärker bevorzugt 8 cm³ oder weniger auf.

Dabei kann der Pflanzstopfen die Form eines Quaders aufweisen, z. B. die Form eines im Wesentlichen seitengleichen Quaders. Der erfindungsgemäße Pflanzstopfen ist jedoch nicht auf diese Form beschränkt. Es ist z. B. ebenfalls denkbar, Pflanzstopfen mit einer zylindrischen oder halbkugeligen Form auszubilden.

Wie oben bereits erläutert, kann das Nährmedium eine wasserunlösliche Komponente umfassen, die eine stabilisierende Wirkung aufweist. Die dortigen Angaben zum Nährmedium sind auf den Pflanzstopfen übertragbar. So kann der Pflanzstopfen ein Nährmedium umfassen, das eine wasserunlösliche Komponente umfasst, wobei sich das äußere Volumen des Pflanzstopfens bei einem Auflösen und Entfernen der Geliermittel Agar und Carrageen aufgrund einer stabilisierenden Wirkung der wasserunlöslichen Komponente um weniger als 60% reduziert.

In einem anderen Aspekt betrifft die Erfindung ein Verfahren zur Herstellung eines Pflanzstopfens, vorzugsweise eines Pflanzstopfens für die automatisierte Kultivierung von Pflanzen, dadurch gekennzeichnet, dass es Schritte umfasst, bei denen man
(a) ein erfindungsgemäßes Nährmedium in flüssigem Zustand bereitstellt;
(b) das Nährmedium in der Form eines Pflanzstopfens geliert; und
(c) optional das Nährmedium sterilisiert.

Das Verfahren dient insbesondere der Herstellung eines erfindungsgemäßen Pflanzstopfens.

In einem Schritt (b) des erfindungsgemäßen Verfahrens wird das Nährmedium in der Form eines Pflanzstopfens geliert. Hierzu kann das in Schritt (a) bereitgestellte flüssige Medium in eine entsprechende Form gefüllt werden. Das Befüllen der Form kann z. B. durch Gießen oder Pumpen geschehen. Die Form kann eine Form mit einer oder mehreren Vertiefungen mit der Form eines Pflanzstopfens sein. Bevorzugt ist die Form mit mehreren Vertiefungen, z. B. 16 oder mehr, 48 oder mehr. Auf diese Weise können Pflanz-Blöcke hergestellt werden, die mehrere Pflanzstopfen umfassen. Die Pflanzstopfen innerhalb der Blöcke sind vorzugsweise zunächst durch geliertes Nährmedium miteinander verbunden, können aber voneinander getrennt werden, z. B. durch Schneiden. Somit richtet sich die Erfindung in einem weiteren Aspekt auf Pflanz-Blöcke, wobei die Pflanzblöcke 16 oder mehr Pflanzstopfen umfassen können, vorzugsweise 48 oder mehr.

Nachdem das Nährmedium in die gewünschte Form gefüllt wurde, wird das zuvor flüssige Nährmedium geliert, insbesondere die in dem Nährmedium enthaltenen Geliermittel Agar und Carrageen. Geeignete Verfahren zur Gelierung von Agar und Carrageen sind Fachleuten bekannt und auch an anderer Stelle hierin beschrieben. Bevorzugt wird das Nährmedium gleichzeitig sterilisiert und geliert. So kann das Nährmedium in Schritt (b) z. B. durch Dampfdrucksterilisation (bei z. B. etwa 121 °C) geliert (und gleichzeitig sterilisiert) werden. In dieser Ausführungsform werden die Schritte (b) der Gelierung und (c) der Sterilisierung gleichzeitig durchgeführt. Alternativ kann die Gelierung aber auch bei geringeren, an anderer Stelle hierin ausgeführten Temperaturen durchgeführt werden. Das Nährmedium nimmt bei dem Gelieren in Schritt (b) die Form eines Pflanzstopfens an, da es sich in der entsprechenden Form befindet.

Während die Schritte (b) und (c) wie oben beschrieben gleichzeitig ausgeführt werden können, ist es auch möglich die Schritte nacheinander durchzuführen. Dabei kann Schritt (c), je nach Auswahl des Sterilisationsverfahrens, vor oder nach dem Gelieren in Schritt (b) durchgeführt werden. Es ist, z. B. möglich Schritt (c) mit Hilfe einer Sterilfiltration vor Schritt (b) durchzuführen oder mit Hilfe einer Mikrowellsterilisation, Dampfdrucksterilisation oder Bestrahlung nach Schritt (b) durchzuführen. Ist es nicht erforderlich, dass der Pflanzstopfen steril ist, so kann Schritt (c) auch weggelassen werden.

In noch einem weiteren Aspekt betrifft die Erfindung ein Verfahren für die automatisierte Kultivierung von Pflanzen, dadurch gekennzeichnet, dass es Schritte umfasst bei denen man
(i) einen erfindungsgemäßen Pflanzstopfen mit einer Pflanze oder einem Pflanzenteil in Kontakt bringt;
(ii) Pflanzstopfen und Pflanze oder Pflanzenteil gemeinsam unter für die Pflanze geeigneten Wachstumsbedingungen derart kultiviert, dass die Pflanze oder das Pflanzenteil in dem Pflanzstopfen wurzelt und Pflanze oder Pflanzenteil und Pflanzstopfen eine Pflanzeinheit bilden; und
(iii) die Pflanzeinheit automatisiert in
   (iii.1) eine Aussparung in einem größeren Pflanzstopfen überführt;
   (iii.2) eine Zelle in einem Kultivierungstablett überführt; und/oder
   (iii.3) ein Erdsubstrat überführt.

Das erfindungsgemäße Verfahren ist insbesondere zur automatisierten Handhabung der Pflanzstopfen sowie der mit den Pflanzstopfen verbundenen Pflanzen geeignet. Dies bedeutet, dass die Pflanze mittels des Pflanzstopfens automatisch, d.h. maschinell, greifbar und weiterverarbeitbar ist. Die Weiterverarbeitung umfasst beispielsweise das Umsetzen des Stopfens in ein anderes Gefäß oder Substrat. Weitere automatisierte Schritte sind ebenfalls denkbar, wie in den Schritten iii.1 bis iii.3 ausgeführt ist. Die automatisierte Handhabung ist unabhängig von der Bewurzelung der Pflanzen, d.h. unabhängig von dem Bewurzelungsstadium der Pflanzen.

Das Inkontaktbringen von Pflanze bzw. Pflanzenteil und Pflanzstopfen umfasst das bloße Auflegen, Andrücken und/oder Einbringen von Pflanze bzw. Pflanzenteilen in den Pflanzstopfen. Bevorzugt wird der Stängel der Pflanze oder des Pflanzenteils leicht in den Pflanzstopfen eingedrückt. Jedenfalls ist der in Schritt (i) hergestellt Kontakt ausreichend fest dafür, dass die Pflanze oder das Pflanzenteil beginnen kann, in dem Pflanzstopfen Wurzeln zu bilden. Der Begriff "Pflanzenteil" bezeichnet dabei insbesondere Pflanzenklone, die durch Trennung einer parentalen Pflanze erzeugt wurden.

Anschließend werden die Pflanze bzw. das Pflanzenteil mit dem Pflanzstopfen gemeinsam kultiviert. Dabei kommt es zu Wachstumsprozessen, insbesondere der Bildung von Wurzeln und eventuell Seitensprossen, sowie zum Wachstum von Blättern und oberen Sprossteilen. Geeignete Wachstumsbedingungen sind Fachleuten bekannt und von der Art der zu kultivierenden Pflanze abhängig. Eine Pflanzeinheit entsteht dabei bereits durch die ersten Wurzeln, welche die Pflanze in dem Pflanzstopfen ausbildet. Insbesondere ist die Pflanzeinheit dadurch gekennzeichnet, dass der Zusammenhalt zwischen Pflanze/Pflanzenteil und Pflanzstopfen fester ist als unmittelbar nach dem Aufbringen / Inkontaktbringen der beiden.

Die so geformte Pflanzeinheit kann auf unterschiedliche Weise weiter verwendet werden. Sie kann direkt in ein anderes Kultivierungselement, z. B. in eine Aussparung in einem größeren Pflanzstopfen oder in ein Erdsubstrat, überführt werden, oder in eine Zelle auf einem Kultivierungstablett überführt werden. Diese kann zum Transport und/oder zur kurzeitigen Lagerung dienen.

Schließlich betrifft die Erfindung in einem weiteren Aspekt auch die Verwendung eines erfindungsgemäßen Nährmediums und/oder Pflanzstopfens für die Kultivierung von Pflanzen, vorzugsweise für die automatisierte Kultivierung von Pflanzen.

Die Erfindung betrifft auch eine Pflanzeinheit bestehend aus einem erfindungsgemäßen Pflanzstopfen und einer oder mehrerer darin verwurzelter Pflanzen, wobei die Pflanzen dabei vorzugsweise in der Gewebekulturphase III (Stage III) vorliegen.

### Kurze Beschreibung der Figuren

In den Zeichnungen sind Ausführungsbeispiele der Erfindung schematisch dargestellt. Es zeigen:

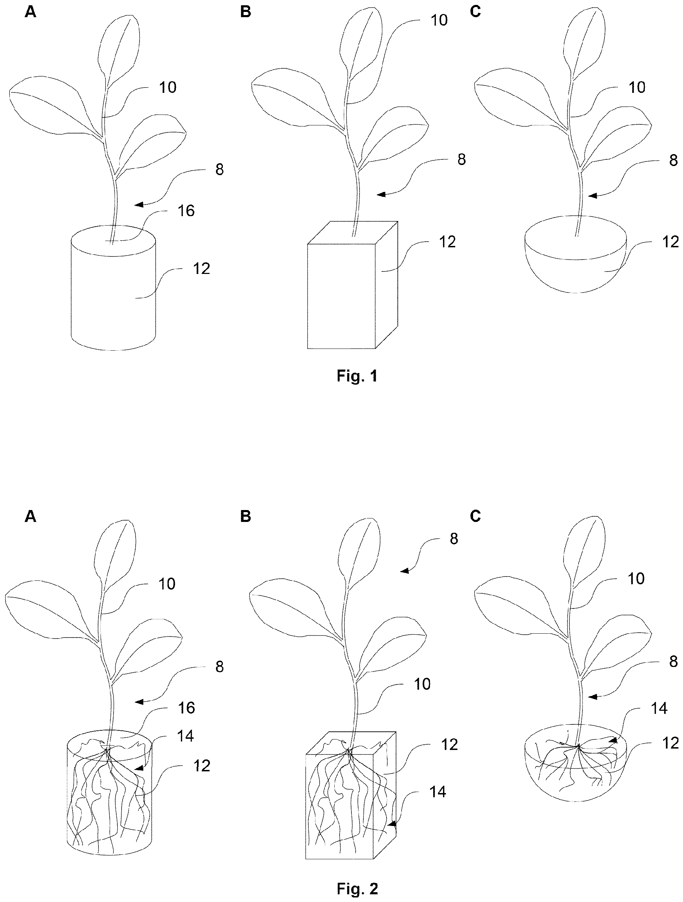

- Fig. 1: verschiedene Ausführungsformen des erfindungsgemäßen Pflanzstopfens (12), die mit einem Spross (10) eine Pflanzeinheit (8) bilden, wobei der Pflanzstopfen (12) eine zylindrische (A), Quader- (B) bzw. Halbkugel- (C) Form aufweist. In der in Fig. 1(A) gezeigten Ausführungsform wurde ein Pflanzschlitz (16) in den Pflanzstopfen (12) eingebracht, in den der Spross (10) eingesteckt wurde. Die Sprosse (10) in Fig. 1(B) und (C) wurden leicht in die Oberfläche des Pflanzstopfens (12) eingedrückt.
- Fig. 2: die Pflanzeinheiten (8) nach Fig. 1, wobei der Spross (10) ein Wurzelwerk (14) in den jeweiligen Pflanzstopfen (12) ausgebildet hat.

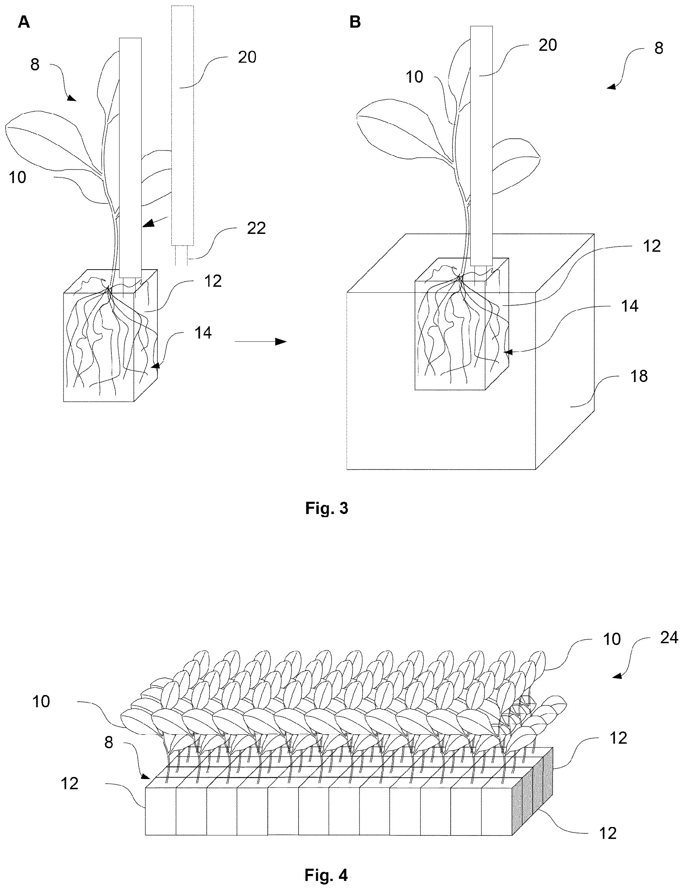

- Fig. 3: den automatischen Transfer einer erfindungsgemäßen, verwurzelten Pflanzeinheit (8) (vgl. Fig. 3(A)), wobei die Pflanzeinheit (8) mittels eines Greifers (20) in eine Aussparung in einen größeren, nicht erfindungsgemäßen Pflanzstopfen eingesetzt wurde, wobei der größere Pflanzstopfen aus einem Erdsubstrat (18) besteht (vgl. Fig. 3(B)). Der Greifer (20) umfasst einen Greiferkopf (22) mit zwei Klingen oder Lanzen, wobei durch Einstechen der Klingen oder Lanzen in den Pflanzstopfen (12) eine derart gute Verbindung zwischen Greifer (20) und Pflanzstopfen (12) geschaffen wird, dass die Pflanzeinheit (8) mittels des Pflanzstopfens transportiert werden kann.
- Fig. 4: einen erfindungsgemäßen Pflanzblock (24) bestehend aus 48 Pflanzeinheiten (8) nach Fig. 1(B).

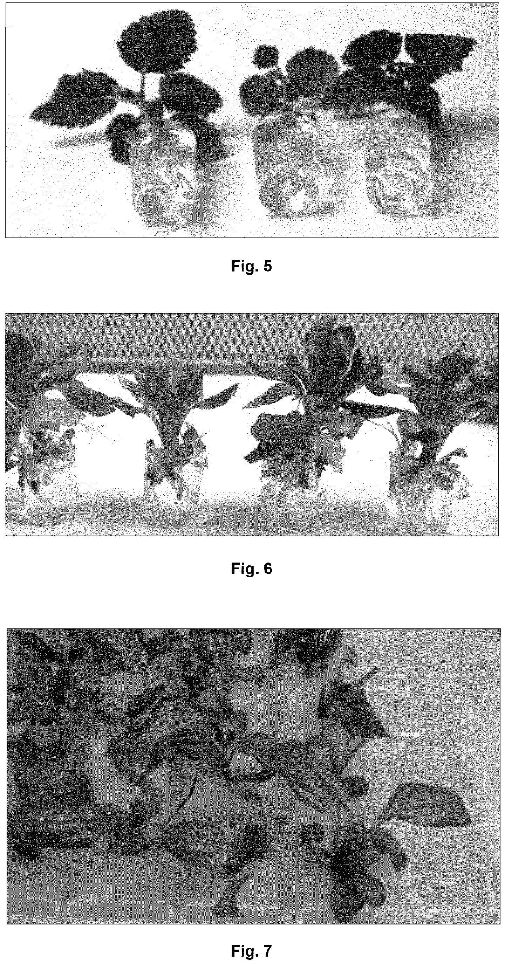

- Fig. 5: Beispiele nicht erfindungsgemäßer Pflanzstopfen ohne eine formstabilisierende Komponente mit verwurzelten *Amelanchier* (Felsenbirnen);
- Fig. 6: Beispiele nicht erfindungsgemäßer Pflanzstopfen ohne eine formstabilisierende Komponente mit verwurzelten *Auricula* (Primel);
- Fig. 7: Beispiele erfindungsgemäßer Pflanzstopfen mit Polyurethanschaum BVB Sublime als formstabilisierender Komponente mit verwurzelten *Echinacea* (Sonnenhüte);

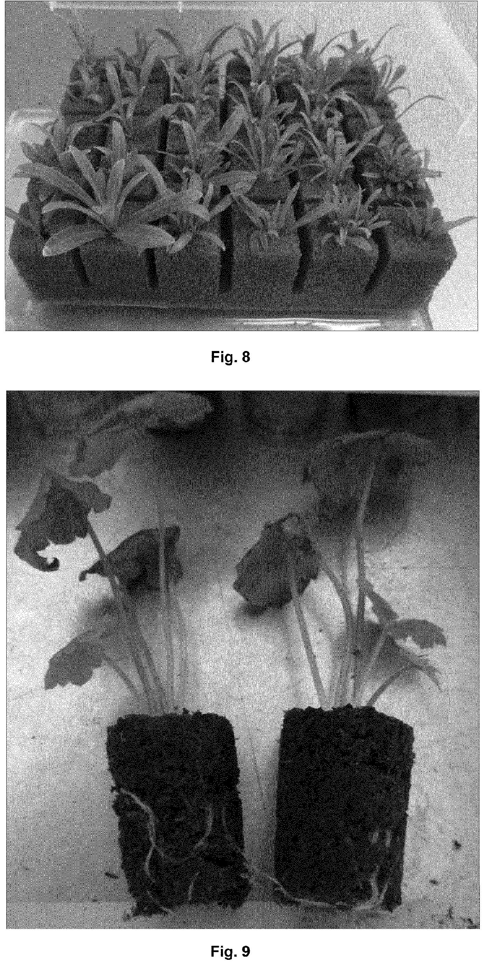

- Fig. 8: Beispiele erfindungsgemäßer Pflanzstopfen mit Polyurethanschaum Oasis als formstabilisierender Komponente mit verwurzelten *Leontopodium* (Edelweiß); und
- Fig. 9: Beispiele erfindungsgemäßer Pflanzstopfen mit Polyurethanschaum und Kokosfasern als formstabilisierenden Komponenten mit verwurzelten *Heuchera* (Purpurglöckchen).
Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

### BEISPIELE

### Beispiel 1: Herstellung eines Pflanzstopfens mit Luffa als formstabilisierender Komponente

### Technische Bedingungen zur Herstellung eines erfindungsgemäßen Pflanzstopfens

Die einzelnen chemischen Komponenten (Makro-, Mikro-, Vitaminkomponenten) wurden in aqua dest. gelöst und bis auf 75% der zu kochenden Menge aufgefüllt. Schließlich wurden Zucker und Phytohormone entsprechend zugegeben. Entsprechende Konzentrationsangaben für die unterschiedlichen chemischen Komponenten wurden der einschlägigen Literatur entnommen (George, Edwin F., Puttock, David J. M., George, Heather J., 1987, Plant Culture Media, Volume I: Formulations and Uses, ISBN 0-9509325-2-3-Vol1, Exegetics Ltd., Edington, Westburgy, Wilts. BA 13 4QG, England).

Nun wurde die zu kochende Menge auf 100% aufgefüllt. Im Anschluss daran wurde der pH-Wert unter Durchrühren des Nährbodens unter tropfenweiser Zugabe von 1N NaOH-Lösung eingestellt. Abschließend wurde die entsprechende Geliermittel-Konzentration zugegeben (6,4 g/l Agar, CERO Agar Agar powder, Type 8925 X; 1,6 g/l Carrageen, CEROGEL Carrageenan (Dänisch Agar Agar), Type 8886, 100% refined kappa-Carageenan). Entweder konnte die Nährbodenrezeptur in nun bereits noch unsterile Trays zum Autoklavieren (121°C, 15 min) abgefüllt werden, oder es erfolgte erst eine Dampfdrucksterilisation (121°C, 15 min), Mikrowellensterilisation, Bestrahlung oder Sterilfiltration unter ständiger Durchmischung und ein Abfüllen in sterile Trays an der Clean-Bench nach der Sterilisation; dazu wurde das Medium auf 50-60°C abgekühlt.

Als wasserunlöslicher, stabilisierender Zuschlagsstoff wurde getrocknete Luffafrucht in Stücke zerteilt, wobei die Stücke die Größe eines Pflanzstopfens aufwiesen. Die Luffastücke wurden dann vor dem Einfüllen des Nährmediums in die zu befüllenden Trays gegeben.

Die sterilen Gefäße mit Trays und den Pflanzstopfen konnten nach Erkalten mit Pflanzen zum Wachstum belegt werden.

### Belegung des Pflanzstopfens mit Pflanzen

In diesem Schritt wurden die Pflanzen entweder manuell an der sterilen Werkbank nach einer Teilung und eventuellem Einkürzen von Blättern mit Skalpell und Pinzette in Form geschnitten oder durch einen Automaten nach Setzmusterprogrammierung mit Laserschnitt bzw. anderer automatisierter Teilung zum weiteren Wachstum bzw. zur Bewurzelung auf den Pflanzstopfen aufgelegt.

### Kulturbedingungen bis zur Weiterverarbeitung der Pflanze im Pflanzstopfen

Die Pflanzen wuchsen nach dem Belegen unter konstanten Bedingungen im künstlich klimatisierten Aufzuchtraum in Gefäßen (Tablett mit Einzelzellen auf Pflanzstopfen). Diese konstanten Bedingungen beinhalteten eine gleichmäßige Temperatur (+/- 1°C), eine gleichmäßige Belichtung mit definierter Lichtfarbe sowie gleichmäßige relative Luftfeuchte und regelmäßigen Luftaustausch.

### Automatischer Pikierprozess des Pflanzstopfens

Die Pflanze wuchs gemäß Kulturprotokoll im Pflanzstopfen im Kulturraum zu einem (bewurzelten) Spross heran. Sie konnte nun automatisch pikiert werden. Die robotische Bearbeitung erfolgte durch Greifen des einzelnen Pflanzstopfens von oben, seitlich und/oder durch Hochdrücken von unten mittels eines Ausdrückmechanismus. Die Greifer können Nadeln, Bleche oder andere Halter sein, sie können aus verschiedenen Materialien gefertigt sein. Der Pflanzstopfen wurde dann mit der Pflanze gegriffen und in einen neuen (größeren) Pflanzstopfen (z. B. unsteriles Erdsubstrat) oder eine Einzelzelle eines Pflanztabletts abgesetzt. Dieser automatische Pikierprozess kann sowohl steril als auch unsteril ablaufen.

### Weiterbehandlung des Pflanzstopfens im Gewächshaus

Das beschriebene *in vitro* Kulturprotokoll endete mit der Adaptation der Pflanze im Pflanzstopfen an die unsterilen Gewächshausbedingungen. Der mit Geliermittel gefestigte und mit einer nicht wasserlöslichen Komponente versehene Pflanzstopfen mit der Pflanze wurzelte in diesem Schritt in einen Erdsubstrat-Stopfen (*Erdsubstrat-Plug).*

### Beispiel 2: Herstellung weiterer Pflanzstopfen

Analog zu dem in Beispiel 1 beschriebenen Verfahren wurden weitere Pflanzstopfen hergestellt und erprobt, in denen kein Luffa als formstabilisierende Komponente eingesetzt wurde. Es wurden die folgenden Pflanzstopfen hergestellt und mit den folgenden Pflanzen getestet:

| **Formstabilisierende Komponente** | **Pflanzenart** | **Ergebnis gezeigt in** |
|---|---|---|
| ohne | Amelanchier | Fig. 5 |
| ohne | Auricula | Fig. 6 |
| Polyurethanschaum (BVB Sublime) | Echinacea | Fig. 7 |
| Polyurethanschaum (Oasis) | Leontopodium | Fig. 8 |
| Polyurethanschaum und Kokosfasern | Heuchera | Fig. 9 |

Die in der Tabelle genannten Polyurethanschäume wurden jeweils in Konzentrationen von 40 bis 70 Gew.-%, bezogen auf den Pflanzstopfen, eingesetzt; die Kokosfasern in Konzentrationen von 5 bis 15 Gew.-%, bezogen auf den Pflanzstopfen.

Gute Ergebnisse (nicht gezeigt) wurden auch mit Gelatineschaum als formstabilisierender Komponente erzielt. In eine Einzelzelle von *in vitro* Trays wurden 4 mg/ml Gelatamp (Roeko) und 2 ml flüssiges Nährmedium (vgl. Beispiel 1) gegeben. Der Gelatineschaum Gelatamp nahm 0,8 ml des Flüssigmediums auf und die resultierenden Pflanzstopfen wiesen eine hinreichende Qualität für die automatische Handhabung auf..

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird.

### Bezugszeichenliste

- 8: Pflanzeinheit
- 10: Spross
- 12: Pflanzstopfen
- 14: Wurzelwerk
- 16: Pflanzschlitz
- 18: Erdsubstrat
- 20: Greifer
- 22: Greiferkopf
- 24: Pflanzblock

## Patentansprüche

1. Nährmedium für die Kultivierung von Pflanzen, umfassend:
(a) 3 g/l bis 18 g/l Agar,
(b) 0,5 g/l bis 3 g/l Carrageen, **dadurch gekennzeichnet, dass** es
(c) eine zusätzliche formstabilisierende Komponente umfasst.

2. Nährmedium nach Anspruch 1, **dadurch gekennzeichnet, dass** die formstabilisierende Komponente eine wasserunlösliche Komponente ist.

3. Nährmedium nach Anspruch 2, **dadurch gekennzeichnet, dass** die wasserunlösliche Komponente ausgewählt ist aus der Gruppe bestehend aus Pflanzenteilen, Kunststoffen, Mineralstoffen und Mischungen derselben und daraus hergestellten Materialien.

4. Nährmedium nach Anspruch 3, **dadurch gekennzeichnet, dass** die Pflanzenteile Jute-, Hanf-, Flachs- und/oder Kokosfasern sind.

5. Nährmedium nach Anspruch 3, **dadurch gekennzeichnet, dass** der Kunststoff Polyurethanschaum ist.

6. Nährmedium nach Anspruch 1, **dadurch gekennzeichnet, dass** die formstabilisierende Komponente eine wasserlösliche Komponente ist, vorzugsweise ausgewählt aus der Gruppe bestehend aus Gelatine und Hydroxyalkylcellulose.

7. Nährmedium nach einem der vorangehenden Ansprüche, **dadurch gekennzeichnet, dass** es Agar in einer Konzentration von 5 g/l bis 12 g/l enthält.

8. Nährmedium nach einem der vorangehenden Ansprüche, **dadurch gekennzeichnet, dass** es Carrageen in einer Konzentration von 1 g/l bis 2,2 g/l enthält.

9. Nährmedium nach einem der vorangehenden Ansprüche, **dadurch gekennzeichnet, dass** das Nährmedium eine Gelstärke von 400 bis 1200 g/cm² aufweist.

10. Pflanzstopfen für die Kultivierung von Pflanzen, vorzugsweise für die automatisierte oder teilweise automatisierte Kultivierung von Pflanzen, **dadurch gekennzeichnet, dass** der Pflanzstopfen ein Nährmedium nach einem der Ansprüche 1 bis 9 umfasst, vorzugsweise aus dem Nährmedium besteht.

11. Pflanzstopfen nach Anspruch 10, **dadurch gekennzeichnet, dass** der Pflanzstopfen ein Volumen von 2 cm³ bis 20 cm³ hat.

12. Pflanzstopfen nach einem der Ansprüche 10 und 11, **dadurch gekennzeichnet, dass** das Nährmedium im Pflanzstopfen eine wasserunlösliche Komponente umfasst und sich das äußere Volumen des Pflanzstopfens bei einem Auflösen und Entfernen der Geliermittel Agar und Carrageen aufgrund einer stabilisierenden Wirkung der wasserunlöslichen Komponente um weniger als 60% reduziert.

13. Verfahren zur Herstellung eines Pflanzstopfens, vorzugsweise eines Pflanzstopfens für die automatisierte oder teilweise automatisierte Kultivierung von Pflanzen, **dadurch gekennzeichnet, dass** es Schritte umfasst, bei denen man
(a) ein Nährmedium nach einem der Ansprüche 1 bis 9 in flüssigem Zustand bereitstellt;
(b) das Nährmedium in der Form eines Pflanzstopfens geliert; und
(c) optional das Nährmedium sterilisiert.

14. Verfahren für die automatisierte oder teilweise automatisierte Kultivierung von Pflanzen, **dadurch gekennzeichnet, dass** es Schritte umfasst bei denen man
(i) einen Pflanzstopfen gemäß einem der Ansprüche 10 bis 12 mit einer Pflanze oder einem Pflanzenteil in Kontakt bringt;
(ii) Pflanzstopfen und Pflanze oder Pflanzenteil gemeinsam unter für die Pflanze geeigneten Wachstumsbedingungen derart kultiviert, dass die Pflanze oder das Pflanzenteil in dem Pflanzstopfen wurzelt und Pflanze oder Pflanzenteil und Pflanzstopfen eine Pflanzeinheit bilden; und
(iii) die Pflanzeinheit automatisiert in
(iii.1) eine Aussparung in einem größeren Pflanzstopfen überführt;
(iii.2) eine Zelle in einem Kultivierungstablett überführt; und/oder
(iii.3) ein Erdsubstrat überführt.

## Claims

1. Growth medium for the cultivation of plants, comprising:
(a) 3 g/l to 18 g/l agar,
(b) 0.5 g/l to 3 g/l carrageenan, **characterized in that** it comprises
(c) an additional shape-stabilizing component.

2. Growth medium according to Claim 1, **characterized in that** the shape-stabilizing component is a water-insoluble component.

3. Growth medium according to Claim 2, **characterized in that** the water-insoluble component is selected from the group consisting of plant parts, plastics, minerals and mixtures thereof and materials produced therefrom.

4. Growth medium according to Claim 3, **characterized in that** the plant parts are jute, hemp, flax and/or coconut fibres.

5. Growth medium according to Claim 3, **characterized in that** the plastic is polyurethane foam.

6. Growth medium according to Claim 1, **characterized in that** the shape-stabilizing component is a watersoluble component, preferably selected from the group consisting of gelatin and hydroxyalkylcellulose.

7. Growth medium according to any of the preceding claims, **characterized in that** it contains agar in a concentration of from 5 g/l to 12 g/l.

8. Growth medium according to any of the preceding claims, **characterized in that** it contains carrageenan in a concentration of from 1 g/l to 2.2 g/l.

9. Growth medium according to any of the preceding claims, **characterized in that** the growth medium has a gel strength of from 400 to 1200 g/cm².

10. Plant plug for the cultivation of plants, preferably for the automated or semiautomated cultivation of plants, **characterized in that** the plant plug comprises a growth medium according to any of Claims 1 to 9 and preferably consists of the growth medium.

11. Plant plug according to Claim 10, **characterized in that** the plant plug has a volume of from 2 cm³ to 20 cm³.

12. Plant plug according to either of Claims 10 and 11, **characterized in that** the growth medium in the plant plug comprises a water-insoluble component and the outer volume of the plant plug is reduced by less than 60% in the case of a dissolution and removal of the gelling agents agar and carrageenan owing to a stabilizing action of the water-insoluble component.

13. Method for producing a plant plug, preferably a plant plug for the automated or semiautomated cultivation of plants, **characterized in that** it comprises steps in which
(a) a growth medium according to any of Claims 1 to 9 is provided in a liquid state;
(b) the growth medium is gelled in the shape of a plant plug; and
(c) optionally the growth medium is sterilized.

14. Method for the automated or semiautomated cultivation of plants, **characterized in that** it comprises steps in which
(i) a plant plug according to any of Claims 10 to 12 is contacted with a plant or a plant part;
(ii) plant plug and plant or plant part are cultivated together under growth conditions suitable for the plant such that the plant or the plant part roots in the plant plug and a plant unit is formed by plant or plant part and plant plug; and
(iii) the plant unit is transferred in an automated manner into (iii.1) a recess in a larger plant plug; (iii.2) a cell in a cultivation tray; and/or (iii.3) a soil substrate.

## Revendications

1. Milieu nutritif pour la culture de plantes, comprenant :
(a) 3 g/l à 18 g/l de gélose,
(b) 0,5 g/l à 3 g/l de mousse d'Irlande, **caractérisé en ce qu'**il comprend
(c) un composant supplémentaire à stabilisation de forme.

2. Milieu nutritif selon la revendication 1, **caractérisé en ce que** le composant à stabilisation de forme est un composant insoluble dans l'eau.

3. Milieu nutritif selon la revendication 2, **caractérisé en ce que** le composant insoluble dans l'eau est choisi dans le groupe consistant en les parties de plantes, les plastiques, les substances minérales et les mélanges de ceux-ci, et les matériaux fabriqués à partir d'eux.

4. Milieu nutritif selon la revendication 3, **caractérisé en ce que** les parties de plantes sont des fibres de jute, de chanvre, de lin et/ou de coco.

5. Milieu nutritif selon la revendication 3, **caractérisé en ce que** le plastique est une mousse de polyuréthane.

6. Milieu nutritif selon la revendication 1, **caractérisé en ce que** le composant à stabilisation de forme est un composant soluble dans l'eau, de préférence choisi dans le groupe consistant en la gélatine et l'hydroxyalkylcellulose.

7. Milieu nutritif selon l'une des revendications précédentes, **caractérisé en ce qu'**il contient la gélose à une concentration de 5 g/l à 12 g/l.

8. Milieu nutritif selon l'une des revendications précédentes, **caractérisé en ce qu'**il contient la mousse d'Irlande à une concentration de 1 g/l à 2,2 g/l.

9. Milieu nutritif selon l'une des revendications précédentes, **caractérisé en ce que** le milieu nutritif présente une force en gel de 400 à 1 200 g/cm².

10. Cube de démarrage pour la culture de plantes, de préférence pour la culture automatisée ou partiellement automatisée de plantes, **caractérisé en ce que** le cube de démarrage comprend un milieu nutritif selon l'une des revendications 1 à 9, de préférence est constitué du milieu nutritif.

11. Cube de démarrage selon la revendication 10, **caractérisé en ce que** le cube de démarrage a un volume de 2 cm³ à 20 cm³.

12. Cube de démarrage selon l'une des revendications 10 et 11, **caractérisé en ce que** le milieu nutritif comprend dans le cube de démarrage un composant insoluble dans l'eau, et le volume extérieur du cube de démarrage se réduit de moins de 60 % dans le cas d'une dissolution et d'une élimination des agents de gélification gélose et mousse d'Irlande, en raison de l'effet stabilisant du composant insoluble dans l'eau.

13. Procédé de fabrication d'un cube de démarrage, de préférence d'un cube de démarrage pour la culture automatisée ou partiellement automatisée de plantes, **caractérisé en ce qu'**il comprend des étapes dans lesquelles
(a) on met à disposition un milieu nutritif selon l'une des revendications 1 à 9 à l'état liquide ;
(b) on gélifie le milieu nutritif sous forme d'un cube de démarrage ; et
(c) éventuellement, on stérilise le milieu nutritif.

14. Procédé de culture automatisée ou partiellement automatisée de plantes, **caractérisé en ce qu'**il comprend des étapes dans lesquelles
(i) on met en contact un cube de démarrage selon l'une des revendications 10 à 12 avec une plante ou une partie de plante ;
(ii) on cultive ensemble le cube de démarrage et la plante ou la partie de plante, dans des conditions de croissance convenant à la plante, de telle sorte que la plante ou la partie de plante forme des racines dans le cube de démarrage, et que la plante ou la partie de plante et le cube de démarrage forment une unité de plantation ; et
(iii) on transfère d'une manière automatisée l'unité de plantation dans
(iii.1) un évidement aménagé dans un cube de démarrage plus grand ;
(iii.2) une cellule dans une table de culture ; et/ou
(iii.3) dans un substrat terreau.
